# EUROPEAN PATENT APPLICATION

(11) **EP 3 804 750 A1**
(43) Date of publication of application: **14.04.2021**
(21) Application number: 19202397.6
(22) Date of filing: 10.10.2019
(51) Int. Cl.: A61K 39/12, A61K 39/29, A61K 47/54, C07K 14/005, A61K 38/00

(54) **CONJUGATE COMPOUNDS FOR PREVENTING AND/OR TREATING HBV AND/OR HDV INFECTIONS, LIVER DISEASES AND FOR TARGETING NTCP**

(71) Applicant: Universität Heidelberg, 69117 Heidelberg (DE)
(72) Inventor: Urban, Stephan, 67434 Neustadt/Weinstraße (DE); Mier, Walter, 64625 Bensheim (DE)
(74) Representative: Grahn, Sibylla Maria

(57) **Abstract**

The present invention relates to conjugate compounds which comprise a peptide moiety (a) which is preferably a hydrophobic modified preS-derived peptide of hepatitis B virus or a respective cyclic peptide, and a NTCP substrate moiety (b), which is preferably a bile acid. The present invention further relates to pharmaceutical compositions comprising at least one conjugate compound. The present invention further relates to medical uses of said conjugate compounds and the pharmaceutical compositions, such as in the diagnosis, prevention and/or treatment of a liver disease or condition, and/or in the inhibition of HBV and/or HDV infection. The present invention further relates to methods of diagnosis, prevention and/or treatment of a said diseases and/or infections.

## Description

The present invention relates to conjugate compounds which comprise a peptide moiety (a) which is preferably a hydrophobic modified preS-derived peptide of hepatitis B virus or a respective cyclic peptide, and a NTCP substrate moiety (b), which is preferably a bile acid. The present invention further relates to pharmaceutical compositions comprising at least one conjugate compound. The present invention further relates to medical uses of said conjugate compounds and the pharmaceutical compositions, such as in the diagnosis, prevention and/or treatment of a liver disease or condition, and/or in the inhibition of HBV and/or HDV infection. The present invention further relates to methods of diagnosis, prevention and/or treatment of a said diseases and/or infections.

### BACKGROUND OF THE INVENTION

Today, about 2 billion people carry serological markers of HBV. About 260 million of them are chronically infected with HBV. According to the Center of Disease Control (CDC) 15-25% of chronically HBV infected people are prone to develop hepatocellular carcinoma (HCC) within a decade if they do not receive appropriate treatment (Shephard *et al*., 2006). HBV-related HCC has a poor prognosis and HBV has therefore been classified by the world health organization (WHO) as the most important naturally occurring human carcinogen. Despite the existence of a prophylactic vaccine, the number of infections will not profoundly decline in the near future due to the lack of curative therapy, the increasing world population and the still limited prophylaxis in the poor countries.

HBV is primarily transmitted via the parenteral route. 90-95% of the acutely infected, immune competent individuals clear the virus, thereby gaining life-long immune protection. About 5-10% of infected people develop chronic Hepatitis B (300,000-500,000 persons in Germany). In contrast, in high endemic areas, particularly Central Africa and Eastern Asia, the main mode of transmission is perinatal from mother to child. Unfortunately, infection of not fully immunocompetent children results in a 90-98% chronic course of the disease.

Hepatitis B-related HCC is therefore the most common malignancy in many of these countries.

Chronic Hepatitis Delta Virus (HDV) infections represent the most severe form of viral hepatitis leading to an even increased rate of cirrhosis and hepatocellular carcinoma, when compared with HBV mono infection. HDV is a satellite virus of Hepatitis B Virus (HBV), which means that patients chronically infected with HDV are always co-infected with HBV. Presently, an estimated 15-25 million people are co-infected with HBV/HDV. However, these numbers are still vague due to the lack of reliable epidemiologic data. Presently, no drugs are approved for treating HDV infected patients. The entry inhibitor Myrcludex B/Bulevirtide has shown promising results in Phase II clinical trials (Mentha *et al*., 2019).

Currently approved therapeutic regiments for the treatment of chronic hepatitis B virus (HBV) infections either address replication steps of the viral genome after an already established infection (Lamivudine, Adefovir, Entecavir, Tenofovir) or act as modulators of the immune system (interferon alpha). Unfortunately, only 10-25% of the patients preserve a sustained virological response upon such therapies. It is therefore of utmost importance to develop novel therapeutics that target so far unaffected replication steps (e.g. virus entry) that may help to eliminate the virus and cure infection.

Despite of the availability of a prophylactic vaccine and reverse transcriptase (RT) inhibitors, the number of HBV-infected people and the number of HBV-related deaths worldwide (presently about 350,000 per year) is increasing. About two thirds of primary liver cancers are attributable to persistent HBV infection (Chan & Sung, 2006).

Specific inhibition of virus entry is an attractive therapeutic concept to control and eventually eliminate acute and chronic infections by different viruses. Entry inhibition in HBV/HDV co-infected patients has shown very promising results in monotherapy (Myr 202-study, see Wedemeyer *et al*., 2017; Wedemeyer *et al*., 2018) including curative potential in a subset of patients when administered in combination with interferon-alpha (Myr 203-study, ClinicalTrials.gov Identifier: NCT03852719, see Wedemeyer *et al*., 2018; Wedemeyer *et al*., 2019).

The human hepatitis B virus (HBV) is a member of the hepadnaviridae. Hepadnaviruses are the smallest enveloped DNA viruses which replicate their DNA genome via reverse transcription of a pgRNA intermediate. During assembly the nucleocapsid acquires three viral envelope proteins termed large (L), middle (M) and small (S). They are encoded in one open reading frame and share the S-domain which is required for membrane anchoring. In addition to the S-domain, M contains an N-terminal hydrophilic extension of 55 amino acids (preS2), while L is further extended by 107, 117 or 118 amino acids (genotype-dependent) termed preS1 (Urban *et al*., 2014). The hepatitis D virus (HDV) is a satellite virusoid utilizing the HBV envelope proteins for entry into hepatocytes. The myristoylated preS1-domain of L plays a key role in HBV and HDV infectivity through a specific interaction with the hepatocyte-specific receptor sodium taurocholate co-transporting polypeptide (NTCP) (Lempp & Urban, 2017).

The inventors have previously identified HBV L-protein derived lipopeptides that block HBV and HDV infection of PHH and HepaRG cells (Gripon *et al*., 2005, Schulze *et al*., 2010, e.g. WO 2009/092611 A1, US 10,323,068). They are derived from the N-terminal 47 amino acids of the preS1-domain of HBV genotype D (HBVpreS/2-48^{myr}) and include the naturally occurring modification with myristic acid. The lead compound is Myrcludex B (see e.g. Bogomolov *et al*., 2016; Blank *et al*., 2016; Wedemeyer *et al,* 2018).

In WO 2009/092612 and WO 2012/107579, whose contents are incorporated herewith by reference in its entirety, the inventors describe hydrophobic modified preS-derived peptides of HBV and their use as vehicles for the specific delivery of compounds to the liver.

The inventors have furthermore identified the receptor responsible for the binding of these HBV L-protein derived lipopeptides, namely sodium taurocholate co-transporting polypeptide (NTCP/SLC10A1) (WO 2014/072526, WO 2014/072524 and WO 2015/014830). See also Ni *et al.* (2014) and Yan *et al.* (2012). In WO 2017/102906 A1, whose contents are incorporated herewith by reference in its entirety, the inventors describe cyclic NTCP-targeting peptides, such as cyclic forms of Myrcludex B, and their uses as entry inhibitors.

It is, thus, an objective of the present invention to provide improved means and methods for the diagnosis, prevention and/or treatment of liver diseases, such as liver diseases related to NTCP-mediated transport.

The present invention further aims to improve the methods and means for the inhibition, prevention and/or treatment of HBV-infection and other HBV-related diseases as present in the prior art and it is, thus, an objective of the present invention to provide improved methods and means which allow for a targeted and effective inhibition, prevention and/or treatment of HBV infection and related diseases.

It is a further objective of the present invention to provide improved means and methods for the inhibition, prevention and/or treatment of HDV infection and HDV-related diseases.

The present invention aims to provide improved means and methods for the inhibition of NTCP as a HBV and HDV receptor and NTCP-mediated transport of natural substrates and further compounds.

### SUMMARY OF THE INVENTION

According to the present invention this object is solved by providing a conjugate compound, comprising
(a) a peptide moiety, and
(b) a NTCP substrate moiety, which addresses the bile acid binding site of sodium taurocholate co-transporting polypeptide (NTCP),
which are covalently attached to each other, preferably via a linker or amino acid side chain(s).

According to the present invention this object is solved by providing a pharmaceutical composition comprising
(i) at least one conjugate compound of the present invention,
(ii) optionally, a pharmaceutically acceptable carrier and/or excipient.

According to the present invention this object is solved by providing the conjugate compound of the present invention or the pharmaceutical composition of the present invention for use in medicine.

According to the present invention this object is solved by providing the conjugate compound of the present invention or the pharmaceutical composition of the present invention for use in the diagnosis, prevention and/or treatment of a liver disease or condition.

According to the present invention this object is solved by a method for the diagnosis, prevention and/or treatment of a liver disease or condition, comprising the administration of a therapeutically effective amount of a conjugate compound of the present invention or a pharmaceutical composition of the present invention.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 21" should be interpreted to include not only the explicitly recited values of 1 to 21, but also include individual values and sub-ranges within the indicated range. Thus, included in this numerical range are individual values such as 1, 2, 3, 4, 5 .... 17, 18, 19, 20, 21 and sub-ranges such as from 2 to 10, 8 to 15, etc. This same principle applies to ranges reciting only one numerical value, such as "at least 90%". Furthermore, such an interpretation should apply regardless of the breadth of the range or the characteristics being described.

### Conjugate compounds

As outlined above, the present invention provides conjugate compounds.

A conjugate compound according to the present invention comprises
(a) a peptide moiety, and
(b) a NTCP substrate moiety, which addresses the bile acid binding site of sodium taurocholate co-transporting polypeptide (NTCP).

Said peptide moiety (a) and NTCP substrate moiety (b) are covalently attached to each other, preferably via a linker or amino acid side chain(s).

### - Peptide moiety (a)

In a preferred embodiment, the peptide moiety (a) is selected from
- a hydrophobic modified preS-derived peptide of hepatitis B virus (HBV) of the general formula I

   **H-[(X)ₘ-P-(Y)ₙ]-R** **(I)**

   wherein
   **P** is the amino acid sequence NPLGFXaaP (SEQ. ID NO: 1), with Xaa being F or L,
   **X** is an amino acid sequence having a length of m amino acids,
      wherein m is 0 or at least 1;
   **Y** is an amino sequence having a length of **n** amino acids,
      wherein n is 0 or at least 1;
      and wherein m + n is 5 to 25, preferably 8 to 20, more preferably 8 to 15;
   **H** is a **hydrophobic modification,** which is
      located N-terminal of P or within X or within Y, and
      selected from acylation and addition of hydrophobic moieties,
   **R** is a **C-terminal modification,**
      which is preferably a moiety that protects from degradation selected from amide, D-amino acid, modified amino acid, cyclic amino acid; natural and synthetic polymer, such as PEG, glycane,
      or
   - a cyclic peptide of the general formula Ia

      **cyclo [(X)ₘ-P-(Y)ₙ]** **(Ia)**

      wherein
      **P, X, Y, m and n** are as defined above,
      and carrying at least one hydrophobic modification at amino acid side chain(s) of X and/or Y, wherein said cyclic peptide is not cyclized within the amino acid sequence of P of SEQ ID NO. 1 and not via amino acid side chains of P,
      wherein the hydrophobic modification is an acylation or addition of hydrophobic moieties,
   or a pharmaceutically acceptable salt thereof.

In one embodiment, the hydrophobic modification of the peptide moiety (a) is an acylation with C8 to C22 fatty acids, such as capric acid (C10), lauric acid (C12), myristoyl (C14), palmitoyl (C16) or stearoyl (C18), preferably myristoyl (C14), palmitoyl (C16) or stearoyl (C18), more preferably myristoyl (C14).

In one embodiment, the hydrophobic moiety or moieties is/are selected from cholesterol, cholesterol derivatives, phospholipids, glycolipids, glycerol esters, steroids, ceramids, isoprene derivatives.

Preferably, the peptide moiety (a) is a peptide of the general formula I, wherein m = 0 to 18 and/or n = 0 to 7, preferably m = 0 to 7 and/or n = 0 to 6 (such as m = 7 and n = 6).

Preferably, the peptide moiety (a) is a cyclic peptide of the general formula Ia, wherein m = 0 to 18 and/or n = 0 to 7, preferably m = 0 to 7 and/or n = 0 to 6, provided that m + n is at least 1.

In an embodiment with
m + n = 0, the peptide has a length of / contains 7 amino acids (namely P);
m + n = 1, the peptide has a length of / contains 8 amino acids;
m + n = 2, the peptide has a length of / contains 9 amino acids;
m + n = 3, the peptide has a length of / contains 10 amino acids;
m + n = 4, the peptide has a length of / contains 11 amino acids;
m + n = 5, the peptide has a length of / contains 12 amino acids;
m + n = 6, the peptide has a length of / contains 13 amino acids;
m + n = 7, the peptide has a length of / contains 14 amino acids;
m + n = 8, the peptide has a length of / contains 15 amino acids;
m + n = 18, the peptide has a length of / contains 25 amino acids;
m + n = 25, the peptide has a length of / contains 32 amino acids.

In a preferred embodiment, the peptide moiety (a) comprises or consists of an amino acid sequence selected from the group of

| | | |
|---|---|---|
| HBVpreS/2-21(B) | GTNLSVPNPLGFFPDHQLDP | SEQ ID NO. 2 |
| HBVpreS/-11-21(B) | GGWSSKPRKGMGTNLSVPNPLGFFPDHQLDP | SEQ ID NO. 3 |
| HBVpreS/2-21(D) | GQNLSTSNPLGFFPDHQLDP | SEQ ID NO. 4 |
| HBVpreS/-11-21(D) | GGWSSKPRKGMGQNLSTSNPLGFFPDHQLDP | SEQ ID NO. 5 |
| HBVpreS/9-16 | NPLGFFPD | SEQ ID NO. 6 |
| HBVpreS/8-16 | PNPLGFFPD | SEQ ID NO. 7 |
| HBVpreS9-17 | NPLGFFPDH | SEQ ID NO. 8 |
| HBVpreS8-17 | PNPLGFFPDH | SEQ ID NO. 9, |

or with the respective modification within P:

| | |
|---|---|
| GTNLSVPNPLGF**L**PDHQLDP | SEQ ID NO. 10, |
| GGWSSKPRKGMGTNLSVPNPLGF**L**PDHQLDP | SEQ ID NO. 11, |
| GQNLSTSNPLGF**L**PDHQLDP | SEQ ID NO. 12, |
| GGWSSKPRKGMGQNLSTSNPLGF**L**PDHQLDP | SEQ ID NO. 13, |
| NPLGF**L**PD | SEQ ID NO. 14, |
| PNPLGF**L**PD | SEQ ID NO. 15, |
| NPLGF**L**PDH | SEQ ID NO. 16, |
| PNPLGF**L**PDH | SEQ ID NO. 17. |

In one embodiment, the peptide moiety (a) comprises further amino acid(s) for covalently attaching the bile acid moiety, wherein said further amino acid(s) are L- or D amino acid(s) and can be natural or non-natural amino acids,
such as lysine (K), D-lysine (k), D-tyrosine (y), cysteine (C), propargylglycine, azidophenylalanine, azidolysine, azidophenylalanine, homoallylglycine, homopropargylglycine, azidohomoalanine, azidonorleucine, azidophenylalanine, propargyloxyphenylalanine and acetylphenylalanine.

In one embodiment, the peptide moiety (a) is a cyclic peptide which comprises further amino acid(s) for cyclization, wherein said further amino acid(s) for cyclization can be natural or non-natural amino acids,
such as cysteine(s) (C), allylglycine, propargylglycine, azidophenylalanine.

### - NTCP substrate moiety (b)

Preferably, the NTCP substrate moiety (b) is selected from
- natural substrate(s) of sodium taurocholate co-transporting polypeptide (NTCP), preferably bile acid(s), bile acid dimer(s) and multimer(s), or
- non-natural substrate(s) of NTCP,
   such as drugs, e.g. ezetimibe, irbesartan, rosiglitazone, zafirlukast, TRIAC, sulfasalazine.

Non-natural substrates are further described in Donkers *et al.* (2017).

Preferably, the the NTCP substrate moiety (b) comprises bile acid(s) which is/are selected from monomeric and polymeric bile acids,
such as
cholate (CA), ursodeoxycholate (UDCA)
taurine- or glycine conjugated bile acids and salts thereof
(taurine- or glycine conjugated dihydroxy and trihydroxy bile salts)
such as
taurocholate (TCA)
glycocholate
taurodeoxycholate (TDCA)
taurochenodeoxycholate (TCDC)
tauroursodeoxycholate (TUDCA)
sulfated bile acids and salts thereof,
- dimers of ursodeoxycholate,
   such as UDCA-UDCA 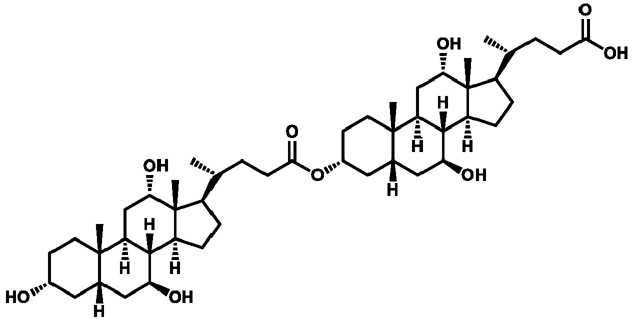 or bis(5β-cholan-24-oic acid 3β-yl)diethyleneglycol (see e.g. Gouin and Zhu, 1996) 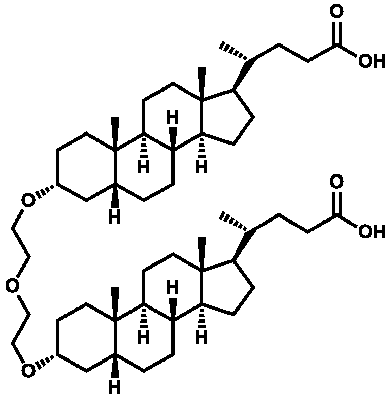
- dimers comprising tauroursodeoxycholate (TUDCA),
   such as CA-TUDCA 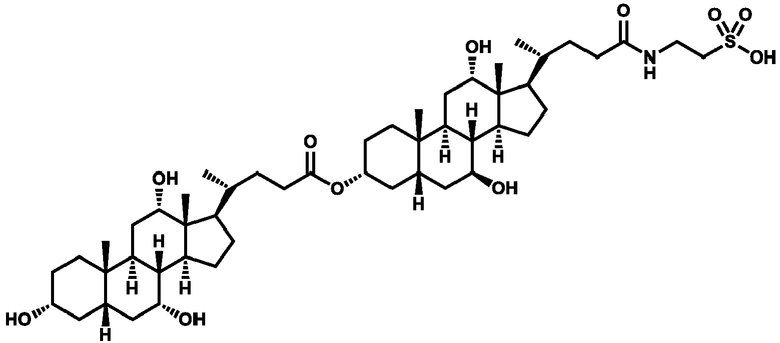
- mixed dimers of the bile acids suc
   h as
   CA-UDCA,

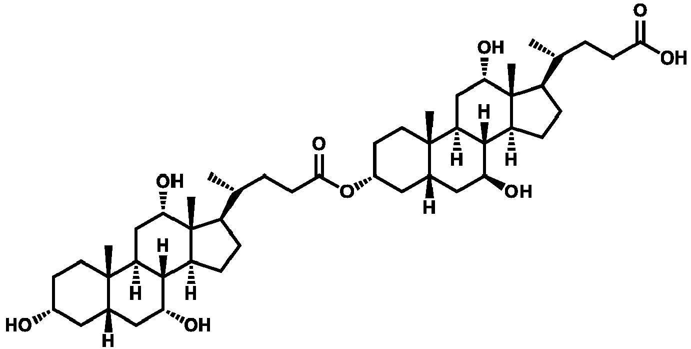

### - Coupling of (a) and (b)

In the conjugate compounds, the peptide moiety (a) and the NTCP substrate moiety (b) are covalently attached to each other, preferably via a linker or amino acid side chain(s).

The coupling site on the bile acid moiety is preferably the carboxylic acid group or the sulfonic acid group of the bile acid moiety.
Coupling is also possible via the ring structure(s) of the bile acid moiety.
Preferably, the bile acid(s) are not attached to the peptide moiety (a) via the 3-hydroxyl group of ring A of the steroid skeleton.

The bile acid moiety (b) is preferably attached to the side chain of a lysine (K). In said embodiment, the peptide moiety can comprise an additional D-amino acid, such as D-Tyrosin (y), to serve as a linker to the lysine.

Preferred conjugate compounds are:
HBVpreS/2-21-K-myr-CA (Genotyp B),
HBVpreS/2-21-K-myr-CA (Genotyp D),
HBVpreS/2-21-K-myr-UDCA (Genotyp B),
HBVpreS/2-21-K-myr-UDCA (Genotyp D),
HBVpreS/2-21-K-myr-(UDCA)2 (Genotyp B),
HBVpreS/2-21-K-myr-(UDCA)2 (Genotyp D),
HBVpreS/2-21-yK-myr-CA (Genotyp B),
HBVpreS/2-21-yK-myr-CA (Genotyp D),
HBVpreS/2-21-yK-myr-UDCA (Genotyp B),
HBVpreS/2-21-yK-myr-UDCA (Genotyp D),
HBVpreS/2-21-yK-myr-(UDCA)2 (Genotyp B),
HBVpreS/2-21-yK-myr-(UDCA)2 (Genotyp D),
HBVpreS/2-21-k-myr-CA (Genotyp B),
HBVpreS/2-21-k-myr-CA (Genotyp D),
HBVpreS/2-21-k-myr-UDCA (Genotyp B),
HBVpreS/2-21-k-myr-UDCA (Genotyp D),
HBVpreS/2-21-k-myr-(UDCA)2 (Genotyp B),
HBVpreS/2-21-k-myr-(UDCA)2 (Genotyp D),
more preferably
HBVpreS/2-21-K-myr-CA (Genotyp B)
HBVpreS/2-21-K-myr-UDCA (Genotyp B)
HBVpreS/2-21-K-myr-(UDCA)2 (Genotyp B).

| ***Conjugate compound*** | ***Hydrophobic modification*** | ***Peptide moiety (a)*** | ***Bile acid moiety (b)*** | ***Coupling of (a) and (b)*** |
|---|---|---|---|---|
| HBVpreS/2-21-K-myr-CA (B) | myr | SEQ ID NO. 2 | CA | K |
| HBVpreS/2-21-yK-myr-CA (B) | myr | SEQ ID NO. 2 | CA | yK |
| HBVpreS/2-21-yK-myr-UDCA (B) | myr | SEQ ID NO. 2 | UDCA | yK |
| HBVpreS/2-21-K-myr-CA (D) | myr | SEQ ID NO. 4 | CA | K |
| HBVpreS/2-21-yK-myr-CA (D) | myr | SEQ ID NO. 4 | CA | yK |
| HBVpreS/2-21-yK-myr-UDCA (D) | myr | SEQ ID NO. 4 | UDCA | yK |

### - Further moieties of the conjugate compounds

The conjugate compound of the present invention can comprise a further moiety or further moieties,
such as
drug(s) or their respective prodrug(s);
tag(s);
label(s), such as fluorescent dye(s), radioisotope(s) and contrast agent(s);
recombinant virus(s);
carrier or depot(s) for drug(s), prodrug(s) or label(s);
immunogenic epitope(s);
hormones;
inhibitor(s);
toxins.

Said further moiety or further moieties is/are preferably covalently attached, such as via a linker, spacer and/or anchor group(s),
e.g. a cleavable linker.

The inventors have found that the conjugate compounds of the present invention are bivalent inhibitors of the entry inhibition is via targeting of the sodium taurocholate cotransporter polypeptide (NTCP/SLC10A1). In particular:
- the peptide moiety of the conjugate allosterically interacts through its essential domain NPLGFXaaP (SEQ. ID NO: 1) with NTCP;
- the NTCP substrate moiety of the conjugate interacts with the bile acid site of NTCP (substrate binding site), since the bile acids are the natural substrates/ligands of NTCP.

Through an extensive mutational analysis the inventors have shown that the essential binding site of the peptide (NPLGF(F/L)P does not directly interact with the bile acid binding site. They have demonstrated that bile acid binding does not exclude the interaction with the essential peptidic part of the new substances. Notably, the blockade of both binding sites resulted in a slower turnover rate of the receptor and therefore prolongs the half life time of the receptor at the surface of hepatocytes. This, and the fixation of the bile salt substrate by the peptide, leads to a synergistic effect of both moieties.

### Pharmaceutical compositions and medical applications

As outlined above, the present invention provides pharmaceutical compositions.

A pharmaceutical composition of the present invention comprises
(i) at least one conjugate compound of the present invention,
(ii) optionally, a pharmaceutically acceptable carrier and/or excipient.
As discussed above, the present invention provides the conjugate compound of the present invention or the pharmaceutical composition of the present invention for use in medicine.

Thus, the conjugate compound(s) of the present invention or the pharmaceutical composition(s) according to the invention are suitable and, thus, provided for the diagnosis, prevention and/or treatment of diseases.

As discussed above, the present invention provides the conjugate compound of the present invention or the pharmaceutical composition of the present invention for use in the inhibition of HBV and/or HDV infection.

As discussed above, the present invention provides the conjugate compound of the present invention or the pharmaceutical composition of the present invention for use in the prevention of a primary HBV and/or HDV infection.

As discussed above, the present invention provides the conjugate compound of the present invention or the pharmaceutical composition of the present invention for use as HBV and/or HDV entry inhibitors

Preferably, HBV infection of any genotype is inhibited or prevented.
Preferably, HDV infection of any genotype of HDV is inhibited or prevented, i.e. HDV with any type of HBV envelope proteins.

Preferably, the entry inhibition is via targeting of the sodium taurocholate cotransporter polypeptide (NTCP/SLC10A1), "NTCP targeting".

As discussed above, in one embodiment, the conjugate compound can further act as a combination inhibitor which
(i) addresses and inhibits NTCP, in particular as a bivalent inhibitor (as discussed above), as well as
(ii) interferes with virus replication via targeting the liver by interaction with NTCP and subsequent affecting the nucleocapsid with its second active domain.

As discussed above, the present invention provides the conjugate compound of the present invention or the pharmaceutical composition of the present invention for use in the diagnosis, prevention and/or treatment of a liver disease or condition.

In one embodiment, the liver disease or condition is selected from hepatitis, cirrhosis, haemochromatosis, preferably hepatitis caused by hepatitis A, B, C, D, E, F, G and H virus or concomitant hepatitis caused by viruses,

In one embodiment, the liver disease or disorder is a disease which involves a liver stadium of a virus or a non-viral pathogen, such as a tropical disease, malaria, schistosomiasis, leishmaniasis, Morbus Wilson.

In one embodiment, the liver disease or disorder is a liver tumor, preferably hepatocellular carcinoma (HCC).

In one embodiment, the liver disease or disorder is a post-transplantation complication after liver transplantation related to bile salt accumulation within the biliary pathway / a post-transplantation-related liver dysfunction.

In one embodiment, the liver disease or condition is related to sodium taurocholate cotransporter polypeptide (NTCP)-mediated transport of compounds into hepatocytes,
or necessitates a delivery of a compound, such as a drug or label, to the site or location of the disease or condition.

Preferably, said liver disease or condition is a liver involved metabolic disease selected from
intrahepatic cholestasis,
poisoning of the liver (by liver toxins) / hepatotoxicity,
drug-induced cholestatic liver disease,
hyperlipidemia,
posthepatic cholestasis,
metabolic syndrome,
non-alcoholic fatty liver disease (NAFLD),
glycogen storage diseases,

Preferably, the compounds which are transported into hepatocytes via NTCP are preferably bile acids, steroids, conjugated and non-conjugated thyroid hormones, liver toxins, compounds that are covalently bound to taurocholate, bromosulphophthalein, drugs.

In one embodiment, the conjugate compound of the present invention or the pharmaceutical composition of the present invention are used in a combination therapy with another therapeutic agent,
such as with
immunomodulatory(s),
e.g. interferon (IFN), interferon lambda,
and/or
agonists or antagonists of RIG-I, MDA-5, or TLRs,
e.g. inarigivir, TLR-7 agonists, TLR-8 agonists.

Recent *in vitro* studies and clinical trials (Myr-203) studies by the investigators have shown that IFNs show a strong synergism with an entry inhibitor. In the case of HDV infections IFNs prevent mitosis mediated cell to cell spread of HDV RNA which cannot be blocked by Myrcludex B. Therefore, administration of both drugs simultaneously shows much stronger suppression of viremia. Furthermore entry inhibition by Myrcludex has been shown to allow the immune modulator IFN to help reconstituting the adaptive immune system (Myr-203 study, see Wedemeyer *et al*., 2019; Wedemeyer *et al*., 2018) and eliminate HBV and HDV. The inventors have identified and described the sodium taurocholate cotransporter polypeptide (NTCP) as target and for use in the prevention and/or treatment of certain liver diseases or conditions, such as liver diseases or conditions that are related to NTCP-mediated transport of compounds (such as bile acids etc.) into hepatocytes, preferably liver involved metabolic diseases (e.g. intrahepatic cholestasis, poisoning of the liver (by liver toxins) / hepatotoxicity, drug-induced cholestatic liver disease, hyperlipidemia, etc.) and cardiovascular diseases. See WO 2014/072524 and PCT/EP2014/066262, which are enclosed herewith in their entirety.

A "liver involved metabolic disease" when used herein refers to metabolic disorders including visceral obesity, diabetes mellitus and dyslipidemia which are influenced by the liver metabolism of lipids and bile acids.

In general, "cholestasis" is a condition where bile constituents cannot be secreted from hepatocytes into the biliary tree or where bile cannot flow from the liver to the duodenum, resulting in hepatocyte bile acid accumulation within hepatocytes.

"Cholestasis" or "intrahepatic cholestasis" when used herein refers to intrahepatic toxic effects of hepatocyte bile acid accumulation related to an insufficient expression and/or activity of bile salt pumps (like BSEP or MRP) in the canalicular membrane.

"Posthepatic cholestasis" when used herein refers to a cholestatic liver disease due to obstruction of the large bile ducts.

"Poisoning of the liver" or "hepatotoxicity" or "toxic liver disease" when used herein refer to toxic effects of drugs independent of bile acid accumulation. These drugs penetrate the hepatocytes via the NTCP-mediated transport and cause several direct toxic effects, by damaging the mitochondria or by activating enzymes in the cytochrome P-450 system leading to oxidative stress.

"Drug-induced cholestatic liver disease" when used herein refers to inhibition of the export of bile acids from hepatocytes due to drug effects on bile salt export pump (BSEP).
Drug-induced cholestasis may be caused by several drugs which inhibit BSEP, such as rifampicin, cyclosporine A, rifamycin SV, bosentan, troglitazone, erythromycin estolate, and glibenclamide (Fattinger *et al*., 2001; Funk *et al*., 2001; Funk *et al*., 2001; Stieger *et al*., 2000; Dawson *et al*., 2012; Morgan *et al*., 2010; Ogimura *et al*., 2011). BSEP is a member of the ATP-binding cassette (ABC) family of transporters (BSEP is also identified as ABCB11) and it is involved in the process of exporting bile acids out of hepatocytes, thus reducing their toxicity to these cells. The above mentioned drugs cause the toxic effects of excess bile acid accumulation because the excretion of bile acid via BSEP is disabled. Inhibition of NTCP-mediated bile acid uptake via the lipopeptide-based compound (such as MyrB) and NTCP counterbalances BSEP inhibition, and thereby prevents hepatotoxicity or is suitable for treatment and/or diagnosis.

"Hyperlipidemia" (or hyperlipoproteinemia, or hyperlipidemia) involves abnormally elevated levels of any or all lipids and/or lipoproteins in the blood.
Hyperlipidemias are divided in primary and secondary subtypes. Primary hyperlipidemia is usually due to genetic causes (such as a mutation in a receptor protein), while secondary hyperlipidemia arises due to other underlying causes such as diabetes. Lipid and lipoprotein abnormalities are common in the general population, and are regarded as a modifiable risk factor for cardiovascular disease due to their influence on atherosclerosis.

"Hypercholesterolemia" (or hypercholesterolaemia) is the presence of high levels of cholesterol in the blood. It is a form of "hyperlipidemia".

"Hyperlipidemia" when used herein preferably refers to hypercholesterolemia which includes elevated LDL cholesterol, reduced HDL cholesterol, elevated triglycerides, clogged arteries leading to high blood pressure, cardiovascular disease (CVD), heart attacks and strokes.

"Metabolic syndrome" refers to a disorder of energy utilization and storage, diagnosed by a co-occurrence of three out of five of the following medical conditions: abdominal (central) obesity, elevated blood pressure, elevated fasting plasma glucose, high serum triglycerides, and low high-density cholesterol (HDL) levels. Metabolic syndrome increases the risk of developing cardiovascular disease, particularly heart failure, and diabetes. Metabolic syndrome is also known as metabolic syndrome X, cardiometabolic syndrome, syndrome X, insulin resistance syndrome, Reaven's syndrome, and CHAOS.

"Non-alcoholic fatty liver disease" (NAFLD) refers to one cause of a fatty liver, occurring when fat is deposited (steatosis) in the liver not due to excessive alcohol use. It is related to insulin resistance and the metabolic syndrome. Non-alcoholic steatohepatitis (NASH) is the most extreme form of NAFLD, and is regarded as a major cause of cirrhosis of the liver of unknown cause.

Preferably, the NTCP-mediated transport is decreased or blocked by the conjugate compounds of the present invention.

As discussed above, in one embodiment, the conjugate compound can act as a combination inhibitor which
(i) addresses and inhibits NTCP, in particular as a bivalent inhibitor (as discussed above), as well as
(ii) interferes with virus replication via targeting the liver by interaction with NTCP and subsequent affecting the nucleocapsid with its second active domain.

### Route of administration

Preferably, the route of administration of the conjugate compounds or pharmaceutical compositions of the present invention is selected from oral, subcutaneous, intravenous, nasal, intramuscular, transdermal, inhalative, by suppository.

A preferred route of administration or application is orally.

A preferred embodiment for nasal administration or application is a nasal spray.

### Therapeutically effective amount

The conjugate compounds or the pharmaceutical compositions of the invention are provided such that they comprise a therapeutically effective amount of said cyclic peptide(s) or of said pharmaceutical composition(s).

A "therapeutically effective amount" of a conjugate compound or a pharmaceutical composition of this invention refers to the amount that is sufficient to inhibit NTCP receptor function. Furthermore, said "therapeutically effective amount" depends on the respective application and desired outcome of inhibition, treatment or vaccination.

Different therapeutically effective amounts are necessary for
(i) antiviral use or entry inhibition (such as 0.01 to 0.5 mg per patient, preferably 0.1 to 1 mg/patient)
   compared to
(ii) uses which require a saturation of NTCP, such as for inhibition of NTCP-mediated transport of e.g. bile acids, drugs etc. (such as 1 mg per patient or 1-2 mg/patient).

In one embodiment, said "therapeutically effective amount" of a conjugate compound or a pharmaceutical composition of this invention refers to the amount that is sufficient to inhibit a HBV and/or HDV infection; prevent a primary HBV and/or HDV infection; treat hepatitis B and/or D and/or vaccinate and/or inhibit entry of HBV and/or HDV *in vivo.*

A preferred therapeutically effective amount is in the range of 10 µg to 1 mg per kg body weight, preferably 10 µg to 100 µg.

Preferably, the therapeutically effective amount is in the range of from about 0.01 mg to about 50 mg per patient and per day, preferably from about 0.1 mg to about 10 mg per patient per day
or is applied to a patient in a dose ranging from 100 nmol per kg to 2µmοl per kg per day / or is applied to a patient in a dose ranging from 10 pmol per kg to 20µmοl per kg body weight.

In case of an IC₅₀ value of the cyclic peptide used of about 10 nM, a preferred therapeutically effective amount is about 100 µg per kg body weight or in the range of 1 to 5 mg per patient. The preferred therapeutically effective amount in the range of 1 to 5 mg per patient can be administered once a day or in other embodiments only once every 2-3 days.

The skilled artisan will be able to determine suitable therapeutically effective amounts.

### Methods of diagnosis, prevention and/or treatment of diseases

As discussed above, the present invention provides a method for the inhibition of HBV and/or HDV infection and/or the prevention of a primary HBV and/or HDV infection.

Said method comprises the administration of a therapeutically effective amount of a conjugate compound of the present invention or a pharmaceutical composition of the present invention. Preferably, HBV infection of any genotype is inhibited or prevented.
Preferably, HDV infection of any genotype of HDV is inhibited or prevented, i.e. HDV with any type of HBV envelope proteins.

As outlined above, the present invention provides a method for the diagnosis, prevention and/or treatment of a liver disease or condition.

Said method comprises the administration of a therapeutically effective amount of a conjugate compound of the present invention or a pharmaceutical composition of the present invention.

In one embodiment, the liver disease or condition is selected from hepatitis, cirrhosis, haemochromatosis, preferably hepatitis caused by hepatitis A, B, C, D, E, F, G and H virus or concomitant hepatitis caused by viruses.

In one embodiment, the liver disease or disorder is a disease which involves a liver stadium of a virus or a non-viral pathogen, such as a tropical disease, malaria, schistosomiasis, leishmaniasis, Morbus Wilson.

In one embodiment, the liver disease or disorder is a liver tumor, preferably hepatocellular carcinoma (HCC).

In one embodiment, the liver disease or disorder is a post-transplantation complication after liver transplantation related to bile salt accumulation within the biliary pathway.

In one embodiment, the liver disease or condition is related to sodium taurocholate cotransporter polypeptide (NTCP)-mediated transport of compounds into hepatocytes,
or necessitates a delivery of a compound, such as a drug or label, to the site or location of the disease or condition,
and preferably is a liver involved metabolic disease selected from
intrahepatic cholestasis,
poisoning of the liver (by liver toxins) / hepatotoxicity,
drug-induced cholestatic liver disease,
hyperlipidemia,
posthepatic cholestasis,
metabolic syndrome,
non-alcoholic fatty liver disease (NAFLD),
glycogen storage diseases,
and wherein the compounds which are transported into hepatocytes via NTCP are preferably bile acids, steroids, conjugated and non-conjugated thyroid hormones, liver toxins, compounds that are covalently bound to taurocholate, bromosulphophthalein, drugs.

In one embodiment, the method comprises a combination therapy with another therapeutic agent,
such as with
immunomodulatory(s),
e.g. interferon (IFN), interferon lambda,
and/or
agonist(s) or antagonist(s) of RIG-I, MDA-5, or TLRs,
e.g. inarigivir, TLR-7 agonists, TLR-8 agonists.

In the methods of the present invention, and as discussed above, the "therapeutically effective amount" depends on the respective application and desired outcome of inhibition, treatment or vaccination.

Different therapeutically effective amounts are necessary for
(i) antiviral use or entry inhibition (such as 0.01 to 0.5 mg per patient, preferably 0.1 to 1 mg/patient)
   compared to
(ii) uses which require a saturation of NTCP (such as 1 mg per patient or 1-2 mg/patient).

In one embodiment, and as discussed above, the therapeutically effective amount is preferably in the range of from about 0.01 mg to about 50 mg per patient, preferably from about 1 mg to about 10 mg per patient,
or wherein the conjugate compound is preferably applied to a patient in a dose ranging from 10 pmol per kg to 20µmοl per kg body weight.

In the methods of the present invention, and as discussed above, the route of administration is preferably selected from oral, subcutaneous, intravenous, nasal, intramuscular, transdermal, inhalative, by suppository.

The following examples and drawings illustrate the present invention without, however, limiting the same thereto.

### BRIEF DESCRIPTION OF THE DRAWINGS

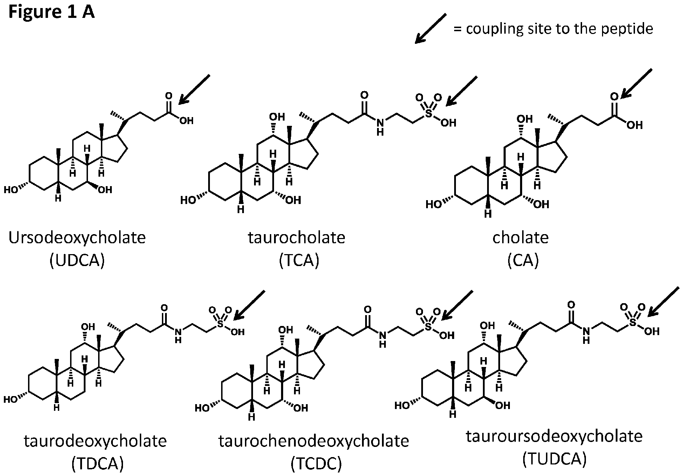

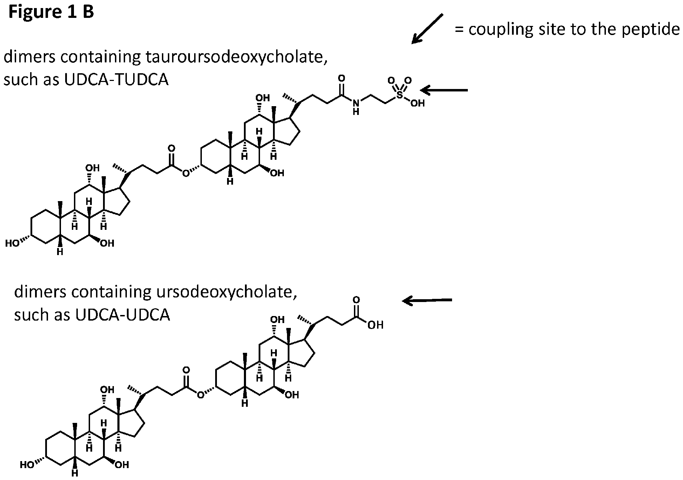

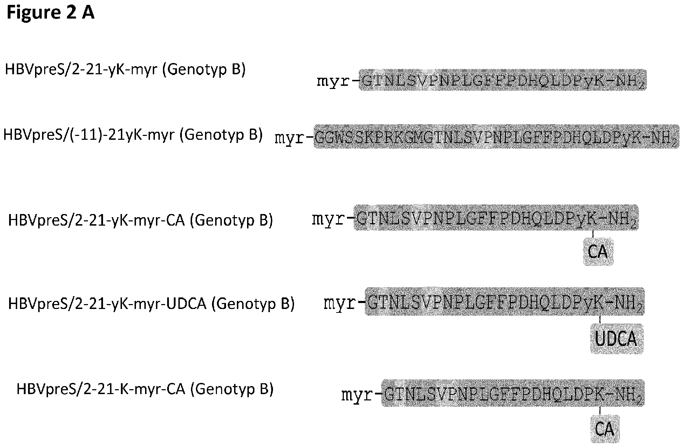

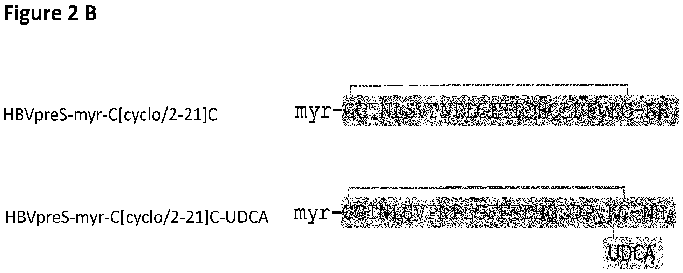

**Figure 1** *Examples of bile acids (for the bile acid moiety (b) of the conjugate compound*
   A, monomers, indicating the preferred coupling site to the peptide moiety,
   B, dimers of bile acids
**Figure 2** *Preferred conjugate compounds.*
   A, peptide moiety is linear,
   B, peptide moiety is a cyclic peptide.
**Figure 3** *HBV*/*HDV infection assay.*
   Tested were conjugate compounds comprising a truncated variant of Myrcludex B (amino acids 2-21) conjugated with bile acids cholic acid (CA) and ursodeoxy cholic acid (UDCA). They were compared with a control peptide not containing a bile acid moiety, namely HBVpreS/(-11)-21^{myr} (Genotype B) and the two bile acids CA and UDCA alone. The conjugate compounds show a significantly higher activity than the peptide alone (1-2 nM versus 20-100 nM).

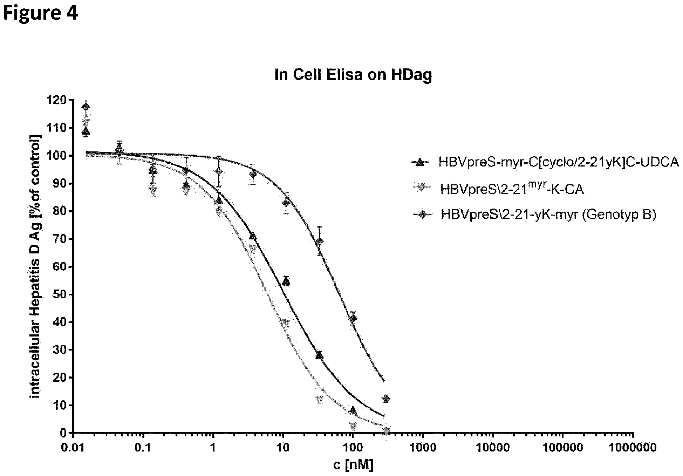

**Figure 4** *HBV*/*HDV infection assay.*
   Tested were conjugate compounds comprising a truncated variant of Myrcludex B (amino acids 2-21) conjugated with cholic acid (CA) and a cyclic peptide (also comprising amino acids 2-21) conjugated with ursodeoxy cholic acid (UDCA). They were compared with a control peptide not containing a bile acid moiety, namely HBVpreS/2-21^{myr} (Genotype B).

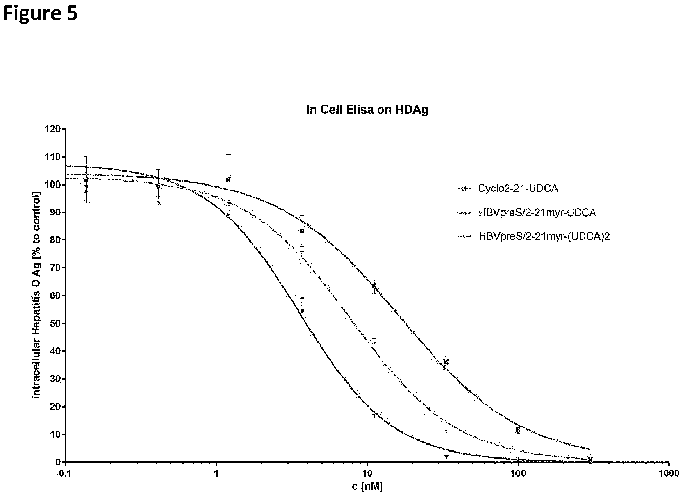

**Figure 5** *HBV*/*HDV infection assay of compounds conjugated with bile acid monomers and dimers.*
   Tested were three compounds: two conjugated with ursodeoxy cholic acid (UDCA) and one with a dimer of ursodeoxy cholic acid (UDCA)2, wherein two comprise amino acids 2-21 of Myrcludex B in a linear form and one as cyclic peptide.

### EXAMPLES

### Example 1 Solid phase synthesis of the peptide moieties

All peptides were synthesized by automated Fmoc/tBu solid phase peptide synthesis on an Applied Biosystems 433A synthesizer as described previously (Schieck *et al*., 2010) Following the peptide synthesis myrictic acid was coupled to the N-terminal end of the peptides. For this purpose the resin was shaken with a solution of myrictic acid (10 eq), HBTU (9.5 eq) and DIPEA (20 eq) in NMP for 2.5 h. The resin was then washed with NMP and DCM.

For the removal of the lysine side chain protective group (Aloc) the resin was incubated with a solution of 3 mg Pd(PPh₃) and 30 mg BH₃NHMe₂ in DCM for 20 min. The resin was washed with DCM and MeOH and then incubated in DCM/MeOH (10:1) two times for 30 minutes. Then it was washed with DCM and Et₂O and dried in vacuo.

### Example 2 Conjugation of the bile acid peptide conjugates

For the coupling of the different bile acids the resin was swollen in NMP. Then a solution of bile acid (10 eq), HBTU (9.5 eg) and DIPEA (20 eq) in NMP was added and shaken for 2.5 h. After this the resin was washed with NMP, DCM and Et₂O and dried under vacuo.
The conjugates were cleaved with 95% TFA/2.5% TIS/2.5% H2O for 1 h and then precipitated in cold Et₂O. Then they were purified by preparative reversed-phase HPLC and the purity was confirmed by analytical HPLC and subsequently analyzed by LC-MS.

### Example 3 HDAg in-cell ELISA (Huh7-hNTCP)

### A. Material:

| | |
|---|---|
| Washing Buffer: | PBS + 0.05% Tween 20 |
| Permeabilization buffer: | PBS + 0.25% Triton X-100 |
| Blocking Buffer: | PBS + 0.05% Tween 20 + 1% Casein |
| White 96-well cell culture plates: | Greiner 655098 |

Advansta ELISABright Chemiluminscence substrate (order via Biozym #541025)
Hydrogen peroxide solution (35%, Sigma 349887)
1ₛₜ Antibody Mouse or rabbit anti HDAg (FD3A7) 1:3000
2nd Antibody: Goat-anti-mouse or goat-anti-rabbit peroxidase conjugated 1:5000

### B. Procedure:

Grow and infect cells in white 96-well plates.
1) Fix cells with 50 µl 4% PFA at RT for 30 min
2) Wash 2x with PBS (not washing buffer)
3) Permeabilize with 100 µl permeabilization buffer at RT for 30 min
4) Block with 100 µl blocking buffer at RT for 30 min
5) Incubate with 100 µl primary antibody (1:3000) diluted in blocking buffer at RT with shaking for 1h
6) 3x 1 min 200µl wash (washing buffer)
7) Incubate with 100µl 3% (HuH7-derived cells) hydrogen peroxide solution (1:12 from stock in PBS for 3%) for 10 min at RT
8) 4x 1 min 200µl wash (washing buffer)
9) Incubate with 100µl 2nd Antibody 1:5000 in blocking buffer at RT with shaking for 1h.
10) 3x 1min 200µl wash (washing buffer) + 2x 10min 200µl wash (washing buffer) + 1x 10min 200µl wash (permeabilization buffer) [VERY IMPORTANT]
11) Add 50µl luminescence substrate (mix according to manufacturer's protocol) and measure at plate reader directly after pipetting.
(Enhanced luciferase protocol, 0.1s measurement)

### B. Results:

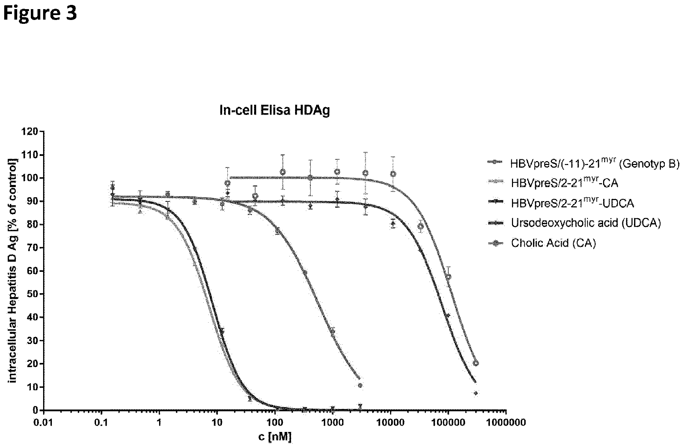

The results are shown in Figures 3, 4 and 5 (genotype B sequence). The following IC50 values were measured:
UDCA: 79 µM
CA: 115 µM
HBVpreS/2-21myr: 62.8 nM
HBVpreS/2-21myr-CA: 7.27 nM
HBVpreS/2-21myr-UDCA: 8.45 nM
Cyclo2-21-UDCA: 10.1 nM
HBVpreS/2-21myr-(UDCA)₂: 3.55 nM.

The features disclosed in the foregoing description, in the claims and/or in the accompanying drawings may, both separately and in any combination thereof, be material for realizing the invention in diverse forms thereof.

### REFERENCES

Blank A, Markert C, Hohmann N, Carls A, Mikus G, Lehr T, Alexandrov A, Haag M, Schwab M, Urban S, Haefeli WE. First-in-human application of the novel hepatitis B and hepatitis D virus entry inhibitor myrcludex B. J Hepatol. 2016 Sep;65(3):483-9.
Bogomolov P, Alexandrov A, Voronkova N, Macievich M, Kokina K, Petrachenkova M, Lehr T, Lempp FA, Wedemeyer H, Haag M, Schwab M, Haefeli WE, Blank A, Urban S. Treatment of chronic hepatitis D with the entry inhibitor myrcludex B: First results of a phase Ib/IIa study. J Hepatol. 2016 Sep;65(3):490-8.
Chan, HL. & Sung, JJ. Hepatocellular carcinoma and hepatitis B virus. Semin Liver Dis 26, 153-161 (2006).
Donkers JM, Zehnder B, van Westen GJP, Kwakkenbos MJ, IJzerman AP, Oude Elferink RPJ, Beuers U, Urban S, van de Graaf SFJ. Reduced hepatitis B and D viral entry using clinically applied drugs as novel inhibitors of the bile acid transporter NTCP. Sci Rep. 2017 Nov 10;7(1):15307. doi: 10.1038/s41598-017-15338-0.
Gripon P, Cannie I, Urban S. Efficient inhibition of hepatitis B virus infection by acylated peptides derived from the large viral surface protein. J Virol 2005;79:1613-1622.
Lempp FA & Urban A. Hepatitis Delta Virus: Replication Strategy and Upcoming Therapeutic Options for a Neglected Human Pathogen. Viruses. 2017 Jul 4;9(7). pii: E172. doi: 10.3390/v9070172. Review.
Mailly L, Xiao F, Lupberger J, Wilson GK, Aubert P, Duong FH, Calabrese D, Leboeuf C, Fofana I, Thumann C, Bandiera S, Lütgehetmann M, Volz T, Davis C, Harris HJ, Mee CJ, Girardi E, Chane-Woon-Ming B, Ericsson M, Fletcher N, Bartenschlager R, Pessaux P, Vercauteren K, Meuleman P, Villa P, Kaderali L, Pfeffer S, Heim MH, Neunlist M, Zeisel MB, Dandri M, McKeating JA, Robinet E, Baumert TF. Clearance of persistent hepatitis C virus infection in humanized mice using a claudin-1-targeting monoclonal antibody. Nat Biotechnol. 2015;33(5):549-54. doi: 10.1038/nbt.3179. Epub 2015 Mar 23.
Gouin-S & Zhu-XX, Synthesis of 3 alpha- and 3 beta-dimers from selected bile acids. Steroids 1996; 61: 664-669.
Mentha N, Clément S, Negro F, Alfaiate D. A review on hepatitis D: From virology to new therapies. J Adv Res. 2019 Mar 29;17:3-15. doi: 10.1016/j.jare.2019.03.009. eCollection 2019 May.
Ni Y, Lempp FA, Mehrle S, Nkongolo S, Kaufman C, Falth M, Stindt J, et al. Hepatitis B and D Viruses Exploit Sodium Taurocholate Co-transporting Polypeptide for Species-Specific Entry into Hepatocytes. Gastroenterology 2014;146:1070-1083.
Schieck, A., Muller, T., Schulze, A., Haberkorn, U., Urban, S., & Mier, W. (2010). Solid-phase synthesis of the lipopeptide Myr-HBVpreS/2-78, a hepatitis B virus entry inhibitor. Molecules, 15(7), 4773-4783. doi:10.3390/molecules15074773
Schulze A, Schieck A, Ni Y, Mier W, Urban S. Fine mapping of pre-S sequence requirements for hepatitis B virus large envelope protein-mediated receptor interaction. J Virol 2010;84:1989-2000.
Shepard, C.W., Simard, E.P., Finelli, L., Fiore, A.E. & Bell, B.P. Hepatitis B virus infection: epidemiology and vaccination. Epidemiol Rev 28, 112-125 (2006).
Urban S, Bartenschlager R, Kubitz R, Zoulim F. Strategies to Inhibit Entry of HBV and HDV into Hepatocytes. Gastroenterology 2014;147(1):48-64.
Wedemeyer H.,.....& Urban S.. 2018. Interim Results of a multicenter, Open-Label Phase 2 Clinical Trial (MYR203) to Assess Safety and Efficacy of Myrcludex B in Combination with Peg-Interferon Alpha 2a in Patients with Chronic HBV/HDV Co-Infection. Hepatology, 68(1):11A.
Wedemeyer H., ....& Urban S. 2019. Final Results of a Multicenter, Open-Label Phase 2 Clinical Trial (MYR203) to Assess Safety and Efficacy of Myrcludex B in Combination with Peg-Interferon Alpha 2a in Patients with Chronic HBV/HDV Co-Infection. J.Hepatology, 70(1).
Wedemeyer H.....& Urban S. et al. 2018. Final results of a multicenter, open-label phase 2b clinical trial to assess safety and efficacy of Myrcludex B in combination with Tenofovir in patients with chronic HBV/HDV co-infection. J Hepatol, 68:S3.
Wedemeyer H et al. 2017. Interim results of a multicenter, open-label phase 2b clinical trial to assess safety and efficacy of Myrcludex B in combination with Tenofovir in patients with chronic HBV/HDV co-infection. Hepatology, (2017), 66 S1, 20A-21A.
Yan H, Zhong G, Xu G, He W, Jing Z, Gao Z, Huang Y, et al. Sodium taurocholate cotransporting polypeptide is a functional receptor for human hepatitis B and D virus. elife. 2012;1:e00049.

## Claims

1. A conjugate compound, comprising
(a) a peptide moiety, and
(b) a NTCP substrate moiety, which addresses the bile acid binding site of sodium taurocholate co-transporting polypeptide (NTCP),
which are covalently attached to each other, preferably via a linker or amino acid side chain(s).

2. The conjugate compound of claim 1, wherein the peptide moiety (a) is selected from
a hydrophobic modified preS-derived peptide of hepatitis B virus (HBV) of the general formula I
**H-[(X)ₘ-P-(Y)ₙ]-R** **(I)**
wherein
**P** is the amino acid sequence NPLGFXaaP (SEQ. ID NO: 1), with Xaa being F or L,
**X** is an amino acid sequence having a length of m amino acids,
wherein m is 0 or at least 1;
**Y** is an amino sequence having a length of **n** amino acids,
wherein n is 0 or at least 1;
and wherein m + n is 5 to 25, preferably 8 to 20, more preferably 8 to 15;
**H** is a **hydrophobic modification,** which is
located N-terminal of P or within X or within Y, and
selected from acylation and addition of hydrophobic moieties,
**R** is a **C-terminal modification,**
which is preferably a moiety that protects from degradation selected from amide, D-amino acid, modified amino acid, cyclic amino acid; natural and synthetic polymer, such as PEG, glycane,
or
a cyclic peptide of the general formula Ia
**cyclo [(X)ₘ-P-(Y)ₙ]** **(Ia)**
wherein
**P, X, Y, m and n** are as defined above,
and carrying at least one hydrophobic modification at amino acid side chain(s) of X and/or Y, wherein said cyclic peptide is not cyclized within the amino acid sequence of P of SEQ ID NO. 1 and not via amino acid side chains of P,
wherein the hydrophobic modification is an acylation or addition of hydrophobic moieties,
or a pharmaceutically acceptable salt thereof.

3. The conjugate compound of claim 1 or 2, wherein the hydrophobic modification of the peptide moiety (a) is an acylation with C8 to C22 fatty acids, such as capric (C10), lauric (C12), myristoyl (C14), palmitoyl (C16) or stearoyl (C18), preferably myristoyl (C14),
or the hydrophobic moiety or moieties is/are selected from cholesterol, cholesterol derivatives, phospholipids, glycolipids, glycerol esters, steroids, ceramids, isoprene derivatives.

4. The conjugate compound of any one of claims 1 to 3, wherein the peptide moiety (a) is peptide of the general formula I, wherein m = 0 to 18 and/or n = 0 to 7, preferably m = 0 to 7 and/or n = 0 to 6 (such as m = 7 and n = 6),
or wherein the peptide moiety (a) is a cyclic peptide of the general formula Ia, wherein m = 0 to 18 and/or n = 0 to 7, preferably m = 0 to 7 and/or n = 0 to 6, provided that m + n is at least 1.

5. The conjugate compound of any of the preceding claims, wherein the peptide moiety (a) comprises or consists of an amino acid sequence selected from the group of SEQ ID Nos. 2 to 17.

6. The conjugate compound of any of the preceding claims, wherein the peptide moiety (a) comprises further amino acid(s) for covalently attaching the bile acid moiety, wherein said further amino acid(s) are L- or D amino acid(s) and can be natural or non-natural amino acids, such as lysine (K), D-lysine (k), D-tyrosine (y), cysteine (C), propargylglycine, azidophenylalanine, azidolysine, azidophenylalanine, homoallylglycine, homopropargylglycine, azidohomoalanine, azidonorleucine, azidophenylalanine, propargyloxyphenylalanine and acetylphenylalanine,
and/or wherein the peptide moiety (a) is a cyclic peptide which comprises further amino acid(s) for cyclization, wherein said further amino acid(s) for cyclization can be natural or non-natural amino acids,
such as cysteine(s) (C), allylglycine, propargylglycine, azidophenylalanine.

7. The conjugate compound of any of the preceding claims, wherein the NTCP substrate moiety (b) is selected from
natural substrate(s) of sodium taurocholate co-transporting polypeptide (NTCP),
preferably bile acid(s), bile acid dimer(s) and multimer(s), or
non-natural substrate(s) of NTCP,
such as drugs, e.g. ezetimibe, irbesartan, rosiglitazone, zafirlukast, TRIAC, sulfasalazine.

8. The conjugate compound of any of the preceding claims, wherein the NTCP substrate moiety (b) comprises bile acid(s) which is/are selected from monomeric and polymeric bile acids,
such as
- cholate (CA), ursodeoxycholate (UDCA)
- taurine- or glycine conjugated bile acids and salts thereof
(taurine- or glycine conjugated dihydroxy and trihydroxy bile salts)
such as
taurocholate (TCA)
glycocholate
taurodeoxycholate (TDCA)
taurochenodeoxycholate (TCDC)
tauroursodeoxycholate (TUDCA)
- sulfated bile acids and salts thereof,
- dimers of ursodeoxycholate,
such as UDCA-UDCA
- dimers comprising tauroursodeoxycholate (TUDCA),
such as 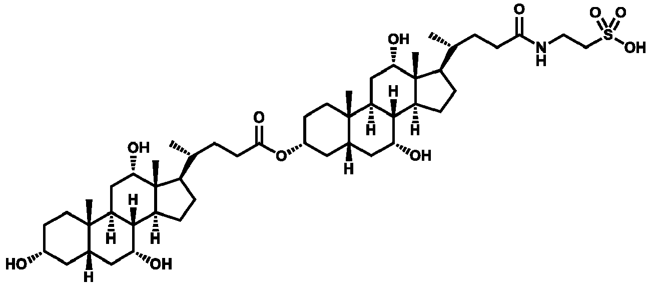
- mixed dimers of the bile acids
such as CA-UDCA,
wherein, preferably, the bile acid(s) are not attached to the peptide moiety (a) via the 3-hydroxyl group of ring A of the steroid skeleton.

9. The conjugate compound of any of the preceding claims, comprising a further moiety or moieties,
such as
drug(s) or their respective prodrug(s);
tag(s);
label(s), such as fluorescent dye(s), radioisotope(s) and contrast agent(s);
recombinant virus(s);
carrier or depot(s) for drug(s), prodrug(s) or label(s);
immunogenic epitope(s);
hormones;
inhibitor(s);
toxins,
preferably covalently attached, such as via a linker, spacer and/or anchor group(s), e.g. a cleavable linker,

10. Pharmaceutical composition comprising
(i) at least one conjugate compound of any of claims 1 to 9,
(ii) optionally, a pharmaceutically acceptable carrier and/or excipient.

11. The conjugate compound of any of claims 1 to 9 or the pharmaceutical composition of claim 10 for use in medicine.

12. The conjugate compound of any of claims 1 to 9 or the pharmaceutical composition of claim 10 for use in the diagnosis, prevention and/or treatment of a liver disease or condition.

13. The conjugate compound or pharmaceutical composition for use according to claim 12, wherein the liver disease or condition is selected from hepatitis, cirrhosis, haemochromatosis, preferably hepatitis caused by hepatitis A, B, C, D, E, F, G and H virus or concomitant hepatitis caused by viruses,
and/or wherein the liver disease or disorder is a disease which involves a liver stadium of a virus or a non-viral pathogen, such as a tropical disease, malaria, schistosomiasis, leishmaniasis, Morbus Wilson,
and/or wherein the liver disease or disorder is a liver tumor, preferably hepatocellular carcinoma (HCC).

14. The conjugate compound or pharmaceutical composition for use according to claim 12 or 13, wherein the liver disease or disorder is a post-transplantation complication after liver transplantation related to bile salt accumulation within the biliary pathway,
and/or wherein the liver disease or condition is related to sodium taurocholate cotransporter polypeptide (NTCP)-mediated transport of compounds into hepatocytes,
or necessitates a delivery of a compound, such as a drug or label, to the site or location of the disease or condition,
and preferably is a liver involved metabolic disease selected from
intrahepatic cholestasis,
poisoning of the liver (by liver toxins) / hepatotoxicity,
drug-induced cholestatic liver disease,
hyperlipidemia,
posthepatic cholestasis,
metabolic syndrome,
non-alcoholic fatty liver disease (NAFLD),
glycogen storage diseases,
and wherein the compounds which are transported into hepatocytes via NTCP are preferably bile acids, steroids, conjugated and non-conjugated thyroid hormones, liver toxins, compounds that are covalently bound to taurocholate, bromosulphophthalein, drugs.

15. The conjugate compound of any of claims 1 to 9 or the pharmaceutical composition of claim 10, comprising a combination therapy with another therapeutic agent,
such as with immunomodulatory(s) and/or agonist(s) or antagonist(s) of RIG-I, MDA-5, or TLRs,
and/or wherein the conjugate compound is administered in a therapeutically effective amount, preferably in the range of from about 0.01 mg to about 50 mg per patient, preferably from about 0.1 mg to about 10 mg per patient,
or is applied to a patient in a dose ranging from 10 pmol per kg to 20µmοl per kg body weight,
and/or wherein the route of administration is selected from oral, subcutaneous, intravenous, nasal, intramuscular, transdermal, inhalative, by suppository.


SEQUENCE LISTING

<110>  Universität Heidelberg

<120>  Conjugate compounds for preventing and/or treating HBV and/or HDV
        infections, liver diseases and for targeting NTCP

<130>  U30951EP

<160>  17

<170>  PatentIn version 3.5

<210>  1
<211>  7
<212>  PRT
<213>  Artificial Sequence

<220>
<223>  Amino acid sequence with Xaa being Phe (F) or Leu (L)


<220>
<221>  misc_feature
<222>  (6)..(6)
<223>  Xaa can be any naturally occurring amino acid

<400>  1

Asn Pro Leu Gly Phe Xaa Pro
1               5


<210>  2
<211>  20
<212>  PRT
<213>  Hepatitis B virus

<400>  2

Gly Thr Asn Leu Ser Val Pro Asn Pro Leu Gly Phe Phe Pro Asp His
1               5                   10                  15


Gln Leu Asp Pro
            20


<210>  3
<211>  31
<212>  PRT
<213>  Hepatitis B virus

<400>  3

Gly Gly Trp Ser Ser Lys Pro Arg Lys Gly Met Gly Thr Asn Leu Ser
1               5                   10                  15


Val Pro Asn Pro Leu Gly Phe Phe Pro Asp His Gln Leu Asp Pro
            20                  25                  30

<210>  4
<211>  20
<212>  PRT
<213>  Hepatitis B virus

<400>  4

Gly Gln Asn Leu Ser Thr Ser Asn Pro Leu Gly Phe Phe Pro Asp His
1               5                   10                  15

Gln Leu Asp Pro
            20

<210>  5
<211>  31
<212>  PRT
<213>  Hepatitis B virus

<400>  5

Gly Gly Trp Ser Ser Lys Pro Arg Lys Gly Met Gly Gln Asn Leu Ser
1               5                   10                  15

Thr Ser Asn Pro Leu Gly Phe Phe Pro Asp His Gln Leu Asp Pro
            20                  25                  30

<210>  6
<211>  8
<212>  PRT
<213>  Hepatitis B virus

<400>  6

Asn Pro Leu Gly Phe Phe Pro Asp
1               5

<210>  7
<211>  9
<212>  PRT
<213>  Hepatitis B virus

<400>  7

Pro Asn Pro Leu Gly Phe Phe Pro Asp
1               5

<210>  8
<211>  9
<212>  PRT
<213>  Hepatitis B virus

<400>  8

Asn Pro Leu Gly Phe Phe Pro Asp His
1               5

<210>  9
<211>  10
<212>  PRT
<213>  Hepatitis B virus

<400>  9

Pro Asn Pro Leu Gly Phe Phe Pro Asp His
1               5                   10


<210>  10
<211>  20
<212>  PRT
<213>  Hepatitis B virus

<400>  10

Gly Thr Asn Leu Ser Val Pro Asn Pro Leu Gly Phe Leu Pro Asp His
1               5                   10                  15

Gln Leu Asp Pro
            20


<210>  11
<211>  31
<212>  PRT
<213>  Hepatitis B virus

<400>  11

Gly Gly Trp Ser Ser Lys Pro Arg Lys Gly Met Gly Thr Asn Leu Ser
1               5                   10                  15

Val Pro Asn Pro Leu Gly Phe Leu Pro Asp His Gln Leu Asp Pro
            20                  25                  30


<210>  12
<211>  20
<212>  PRT
<213>  Hepatitis B virus

<400>  12

Gly Gln Asn Leu Ser Thr Ser Asn Pro Leu Gly Phe Leu Pro Asp His
1               5                   10                  15

Gln Leu Asp Pro
            20


<210>  13
<211>  31
<212>  PRT
<213>  Hepatitis B virus

<400>  13

Gly Gly Trp Ser Ser Lys Pro Arg Lys Gly Met Gly Gln Asn Leu Ser
1               5                   10                  15

Thr Ser Asn Pro Leu Gly Phe Leu Pro Asp His Gln Leu Asp Pro
            20                  25                  30

<210>  14
<211>  8
<212>  PRT
<213>  Hepatitis B virus

<400>  14

Asn Pro Leu Gly Phe Leu Pro Asp
1               5

<210>  15
<211>  9
<212>  PRT
<213>  Hepatitis B virus

<400>  15

Pro Asn Pro Leu Gly Phe Leu Pro Asp
1               5

<210>  16
<211>  9
<212>  PRT
<213>  Hepatitis B virus

<400>  16

Asn Pro Leu Gly Phe Leu Pro Asp His
1               5

<210>  17
<211>  10
<212>  PRT
<213>  Hepatitis B virus

<400>  17

Pro Asn Pro Leu Gly Phe Leu Pro Asp His
1               5                   10